# EUROPEAN PATENT APPLICATION

(11) **EP 4 702 927 A1**
(43) Date of publication of application: **04.03.2026**
(21) Application number: 25194174.6
(22) Date of filing: 05.08.2025
(51) Int. Cl.: A61B 6/03, A61B 6/46, A61B 6/00

(54) **COMPUTED TOMOGRAPHY SIMULATOR FOR ESTIMATING DOSE AND IMAGE QUALITY**

(30) Priority: 30.08.2024 US 202418821570
(71) Applicant: GE Precision Healthcare LLC, Waukesha, WI 53188 (US)
(72) Inventor: RUPCICH, Franco, Waukesha, 53188 (US); THIBAULT, Jean-Baptiste, Waukesha, 53188 (US); LEWIS, Chelsey Amanda, Waukesha, 53188 (US); LONDT, John Howard, Waukesha, 53188 (US); FLEJTER, Matthew, Waukesha, 53188 (US)
(74) Representative: AWA Sweden AB

(57) **Abstract**

A computer-implemented method for a simulated computed tomography (CT) system is disclosed. The method includes receiving, via a user interface, a selected scan protocol and one or more characteristics of a patient or patient group. Using a CT simulator, determining parameter settings for scanning the patient or patient group are determined. The method further includes determining a projected image quality measure and dose using the CT simulator and generating a customized scan protocol for the patient or patient group based on the projected image quality measure and dose.

## Description

### TECHNICAL FIELD

Embodiments of the subject matter disclosed herein relate computed tomography (CT) imaging systems, and in particular, to simulating imaging protocols.

### BACKGROUND

In computed tomography (CT) imaging systems, an electron beam generated by a cathode is directed towards a target within an x-ray tube. A fan-shaped or cone-shaped beam of x-rays produced by electrons colliding with the target is directed towards a subject, such as a patient. After being attenuated by the object, the x-rays impinge upon an array of radiation detector elements. An electrical signal is generated at each detector element, and the electrical signals generated at the detector elements are transmitted to a data processing system for analysis which ultimately produces an image.

During scanning to acquire projection data, it is generally desirable to reduce X-ray dose received by the subject and to increase image quality. Within a CT protocol, there may be numerous scan settings that impact a trade-off between dose and image quality. The scan settings established by the CT protocol may not result in an image of a desired quality, whereby an operator of a CT system may adjust one or more scan settings of the protocol to increase the image quality or reduce the dose. However, determining an optimal combination of scan settings to achieve the desired quality/dose balance may be difficult. As a result, various diagnostic scans may be performed on a patient to achieve a desired image, increasing an amount of radiation to which the patient is exposed.

Additionally, CT users (e.g., medical physicists) optimize and validate the clinical efficacy of protocols via phantom scans. This can be a burdensome and time-consuming process, especially for sites with many protocols. Furthermore, many sites may only have one or two phantoms that do not span a full space of anatomical regions, clinical scenarios, or patient sizes.

### SUMMARY

The present disclosure relates to a simulated computed tomography (CT) system and method for optimizing scan protocols. The method involves receiving a selected scan protocol and one or more characteristics of a patient or patient group via a user interface. Using a CT simulator, determining parameter settings for scanning the patient or patient group are determined. The CT simulator then calculates a projected image quality and dose based on these parameter settings. A customized scan protocol is generated for the patient or patient group based on the projected image quality and dose. The system includes a user interface for receiving the scan protocol and patient characteristics, a CT simulator for determining parameter settings and projecting image quality and dose, a display device for showing the projected image quality and dose, and a memory for storing the customized scan protocol. The system can operate remotely from a CT imaging system and can extract patient characteristics from a scout scan. The user interface allows for confirmation and adjustment of parameter settings to ensure the customized scan protocol meets the desired criteria.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a pictorial view of a simulated computed tomography (CT) imaging system, in accordance with one or more embodiments of the present disclosure;
FIG. 2 shows a more detailed block schematic diagram of the example simulated CT imaging system, in accordance with one or more embodiments of the present disclosure;
FIG. 3 is a flowchart illustrating a method for simulating a diagnostic scan to be performed using a CT imaging system, according to embodiments of the present disclosure;
FIG. 4 is a flowchart illustrating a method for estimating a dose curve as a function of patient size by simulating a protocol for patients of various sizes, according to embodiments of the present disclosure;
FIG. 5 shows an exemplary graphical user interface (GUI) of a CT simulation system, according to embodiments of the present disclosure;
FIG. 6 depicts another exemplary graphical user interface (GUI) or portion of the GUI for a simulated CT system; and
FIG. 7 is a graph comparing a first dose curve showing dose as a function of patient size with a second, similar dose curve estimated using a CT simulation system, according to embodiments of the present disclosure.

### DETAILED DESCRIPTION

This description and embodiments of the subject matter disclosed herein relate to computed tomography (CT) imaging systems. In particular, systems and methods are disclosed for simulating a projected image quality (IQ) measure or a projected dose prior to performing a scan using actual patient data. The patient data may include one or more patient characteristics of a given patient, such as size, which may be used to configure a protocol for the patient or group of patients. In various embodiments, the patient characteristic may be extracted from a CT localizer image, also referred to herein as a scout image. Scan parameters included in the protocol may then be optimized or customized for the patient or group of patients by an operator, where the operator may adjust the scan parameters and simulate a diagnostic scan, to determine a desired IQ/dose balance for performing the diagnostic scan.

In some CT systems, the radiation source and the detector array are rotated with a gantry within the imaging plane and around the object to be imaged such that an angle at which the radiation beam intersects the object constantly changes. A group of radiation attenuation measurements, e.g., projection data, from the detector array at one gantry angle is referred to as a "view." A "scan" of the object includes a set of views made at different gantry angles, or view angles, during one revolution of the radiation source and detector. The term "view" is used to mean one data acquisition whenever there are multiple data acquisitions from different angles.

Typically, to plan an optimal diagnostic scan, a patient undergoes a scout scan. A scout scan is conventionally a two-dimensional (2D) scan of the patient that produces 2D scout projection data. To perform the 3D scout scan, the patient may be positioned within the imaging space of an imaging system. A table of the imaging system may be controlled, for example via the table motor controller, to move such that a region of interest to be imaged is within the bore of the gantry. The imaging system is then controlled to acquire scout projection data with a reduced radiation dosage with respect to a typical diagnostic scan. For example, the scout scan may be performed with a lower tube current and a higher helical pitch relative to a typical diagnostic scan. During the scout scan, the subject may be moved through the gantry bore while the x-ray source is controlled to generate a low-dose x-ray beam. For a higher helical pitch, one or more of the speed of the table movement and the rotational speed of the x-ray source around the gantry bore may be increased relative to a normal scan.

A 2D image (e.g., a scout image) that is similar to a plain radiography image is reconstructed from the 2D scout projection data, which can be used to determine and confirm the general location of particular anatomy or another region of interest prior to performing the full diagnostic scan. Using the scout image, an operator may identify anatomical landmarks to ensure the target anatomy is in the field of view and so that proper techniques may be selected for different areas of the patient. When a scout scan is performed, attenuation data of the patient may be determined and stored in memory of the computing device. After the scout scan is performed, the diagnostic scan may be performed, based on the selected techniques.

The diagnostic scan may be performed in accordance with various scan parameters of the imaging system, which may be set prior to performing the diagnostic scan. Prior to performing the diagnostic scan, the operator may select a desired protocol for performing the scan. In various embodiments, the scan parameters may be generated automatically by one or more AI algorithms based on the selected protocol. One or more scan parameters may be configured based on the scout scan. For example, a size of the patient may be determined from the scout scan, and the patient size may be used to set various scan parameters. The scan parameters determine, among other things, a radiation dose that will be applied to the patient for a desired image quality (IQ) measure of an image reconstructed from projection data acquired during the diagnostic scan. To achieve a higher IQ measure, the radiation dose may be increased. Conversely, if a reduced radiation dose is used, the IQ may be decreased.

Because of this natural trade-off between radiation dose and IQ, the operator may choose to adjust one or more parameter settings prior to performing the diagnostic scan to reduce an exposure of the patient to radiation or to achieve a desired IQ measure. However, determining which parameters to adjust, and determining ideal settings for the adjusted parameters may not be straightforward. An effectiveness of such adjustments towards achieving both a desired dose and a desired IQ measure may depend on operator experience and trial and error.

For example, the operator may perform a diagnostic scan based on the protocol and reconstruct an image with a first IQ measure at a first dose. The image may not be of a desired quality, whereby the operator may make a first set of adjustments to the scan parameters, and perform the diagnostic scan a second time to achieve a reconstructed image with a desired IQ measure at an acceptable dose. The first set of adjustments may not achieve the desired IQ measure, and the operator may perform a second set of adjustments of the scanning parameters, and perform a third diagnostic scan to achieve the desired IQ measure. As a result of performing the three diagnostic scans, the patient is exposed to a higher amount of radiation than desired, to achieve an ideal image. Alternatively, the first set of adjustments may achieve an IQ measure that is more than sufficient to perform a diagnosis, also exposing the patient to more radiation than desired. Due to variance between patients, because a number of parameters that could be adjusted may be large, and because of interactions between the parameters, determining an optimal set of adjustments for achieving a desired dose/IQ balance may be difficult, and may entail performing repeated diagnostic scans.

Further, as a result of difficulties in determining optimal parameter settings for a given type of scan, region of interest, patient, etc., the optimization of standard protocols used on a plurality of patients may include performing a large number of scans on different phantoms, which may be time consuming, and may reduce an availability of the imaging system for performing patient scans, thereby decreasing a functionality and efficiency of the imaging system.

To address these issues, a software-based tool is provided herein that enables a user to preview estimated dose and image quality (IQ) data for a patient or group of patients, based on one or more physical characteristics (e.g., such as size) of the patient and the selected protocol. The one or more patient characteristics may be extracted from a scout image, in various embodiments. The software-based tool simulates a front-end CT scanner console and allows a user to enter in patient data and/or load a previously acquired patient scout DICOM image. Once the scout image or images are loaded, the user can select a desired protocol and simulate scanning the patient in accordance with the selected protocol, based on scout image (and/or the patient data). The system may calculate and display a projected noise index, projected dose data, including a projected CT dose index (CTDIᵥₒₗ), a projected dose-length product (DLP), and a projected exposure duration for the selected protocol, scan range, and scan settings. For features that automatically determine certain scan parameters based on patient size, the user can also see which settings were chosen.

Additionally, the tool enables the user to adjust the scan parameters, and view immediate visual and quantitative dose and IQ feedback on the effect of the adjustments to the scan parameters. Manual changes to scan settings causes the dose and image quality projections to update accordingly. In this way, the user can determine a set of scan parameters for scanning a particular patient or patient group that achieves a desired balance between radiation dose and image quality for the patient, prior to performing a scan on the patient, and without relying on repeated diagnostic scans.

Further, the tool enables the user to obtain accurate projections of IQ measure and dose and visualize predicted system behavior for clinical protocols across a range of anatomical regions and patient sizes from actual patient scouts. Using the tool, local outliers and undesirable behavior may be prevented by choosing appropriate parameters for each portion of the imaging space, before any real patients are imaged at a site. This allows medical physicists to obtain accurate estimates of image quality and dose across many protocols and patient sizes, thereby facilitating more rapid optimization and validation of protocols for a general population. For example, protocol scan settings may be tuned to achieve a target dose or dose range as a function of patient size; achieve a more consistent dose curve as a function of size (e.g., ensuring dose consistently increases with patient size); and/or achieve a consistent image quality as a function of patient size and/or imaging scenario. An output of the tool can be plotted against patient size (as determined from the scout images), to visualize and validate dose and image quality across a range of patient sizes. Doing this over a large number of pre-existing scouts may enable protocol optimization without being limited by scanning phantoms, or slowly building experience with actual patient scanning over time. As a result, a consistent image quality may be generated across similar patients for efficient diagnostic reads, and/or consistent image quality over time for repeat patients, when comparing quantitative measurements as a function of time that would have inherent variability not attributable to a change in disease state if protocol optimization were performed poorly.

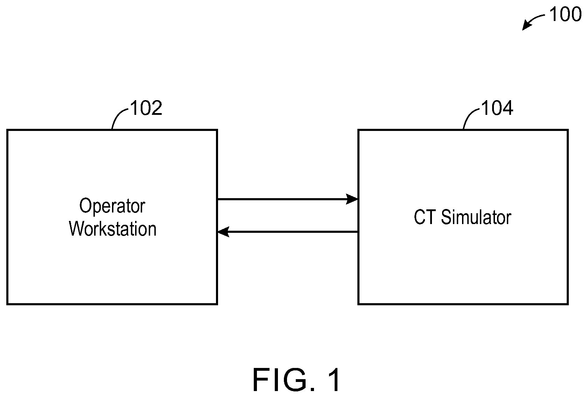

Referring now to the figures, FIG. 1 illustrates an exemplary simulated CT system 100. The example simulated CT system includes an operator workstation 102 and a CT simulator 104 accessible using the operator workstation 102. The example CT simulator 104 may be located on a server in a hospital system, and may be accessible via cloud access. The simulated CT system 100 may be operably/communicatively coupled to one or more user input devices and a display device. For example, user input devices may include a keyboard and a mouse or similar device coupled to a computer, and display device may include a monitor coupled to the computer. Other input devices may be included in place of or in addition to a mouse and keyboard, including but not limited to, a touchscreen, a trackpad, a microphone, and a camera. The input devices allow an operator (e.g., medical physicist) to select one or more inputs to the CT simulator 104. The inputs may include a patent's electronic medical record or history, previous patient scans, such as scout scan and/or diagnostic images, and a library of other images that related to a patient's symptoms and/or physician's instructions. The CT simulator 104 processes these inputs with respect an imaging protocol selected. The CT simulator then outputs an optimized scan protocol, including the parameter settings for the scan protocol for performing scout and/or diagnostic scans for a patient or patient group. For example, a scout scan for a large adult patient may be input and a protocol for a chest scan may be selected via the operator workstation. The CT simulator 104 can simulate multiple variations of parameters for the protocol to determine the optimal parameters that balance an acceptable IQ measure with a dose to the patient. The output parameters can then be used on a CT imaging system for a scout and/or diagnostic scan for a large adult patient needing a chest scan. In some examples, scout scans for multiple sizes of patients can be selected simultaneously, and the CT simulator 104 can output parameters for each patient group.

The simulated CT system 100 includes a processor configured to execute machine readable instructions stored in non-transitory memory. The processor may be single core or multi-core, and the programs executed thereon may be configured for parallel or distributed processing. In some embodiments, the processor may optionally include individual components that are distributed throughout two or more devices, which may be remotely located and/or configured for coordinated processing. In some embodiments, one or more aspects of the processor may be virtualized and executed by remotely-accessible networked computing devices configured in a cloud computing configuration. Non-transitory memory may store one or more simulated CT scanner software applications, which may include software applications mirroring those installed in an operator console of a CT imaging system and/or on the operator workstation 102 and used to operate a CT scanner to acquire projection data during a scan and reconstruct an image based on the acquired projection data.

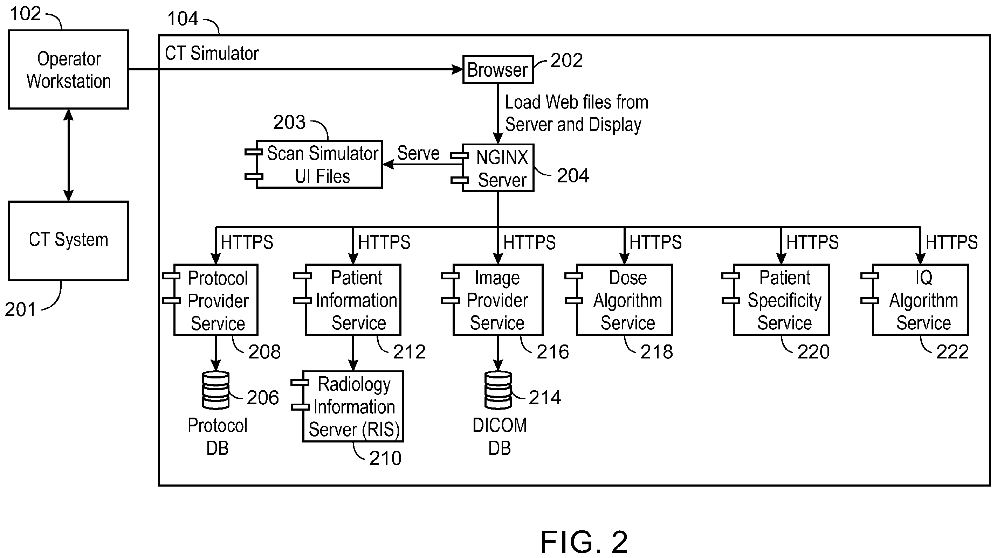

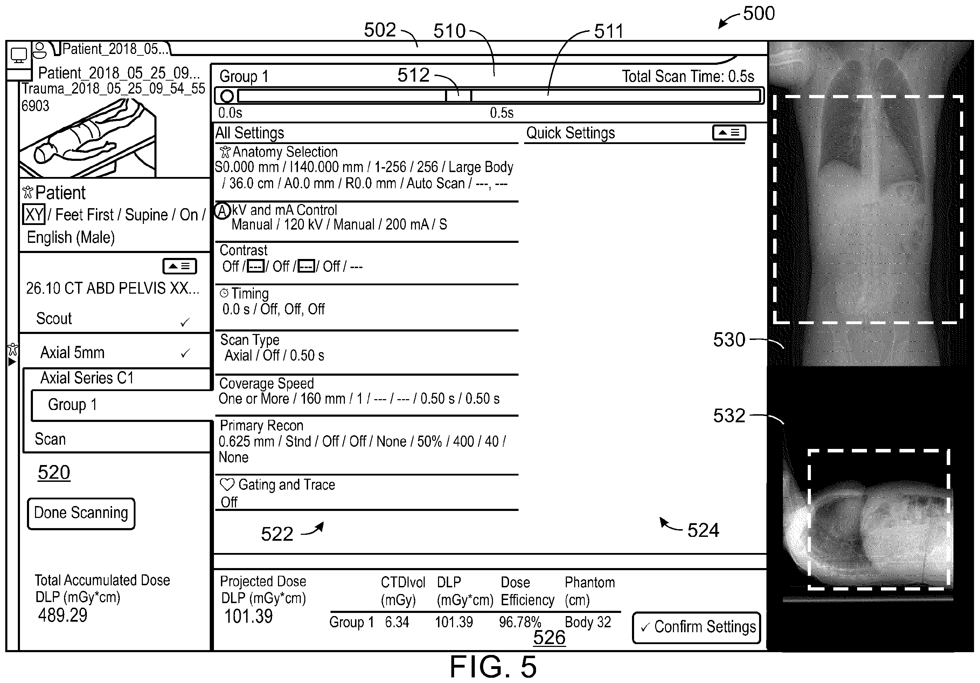

FIG. 2 depicts a more detailed block diagram of the example CT imaging system of FIG. 1. The simulated CT system 100 may be a stand-alone system, meaning, simulated CT system 100 may not be a part of or coupled to an imaging system 201. In various embodiments, simulated CT system 100 may be installed on a computing device of a radiologist or other user. For example, a portion of simulated CT system may be disposed at a device (e.g., edge device, server, etc.) communicably coupled to imaging system 201 via wired and/or wireless connections. The example CT simulator 104 includes an internet browser 202. The internet browser 202 enables the user to access the CT simulator 104 from the operator workstation 102. The internet browser 202 enables access to a server 204. The server accesses scan simulator user interface (UI) files 203, which includes an interface to allow the user to interact with the CT simulator. An example user interface is depicted in FIG. 5, and may include areas for input or prompts to be selected by the user and areas for output of information.

Via the server 204, the CT simulator 104 is able to access a protocol database 206 via a protocol provider service 208. The protocol database 206 stores existing protocols from which the user may select a protocol to optimize using the CT simulator 104. As discussed in conjunction with FIG. 1, the user may select a protocol, such as a protocol for a chest scan, from the protocol database 206 using the input device of the operator workstation.

Additionally, the user may access a radiology information server 210 via a patient information service 212 and the server 204. The radiology information server may include patient data available to the hospital system. For example, patient data may include a patient's medical record, which may include height, weight, demographic information, etc. Patient data may also include previous patient scans (e.g., scout scans, diagnostic scans), and diagnostic information (e.g., information related to a diagnosis of a patient, e.g., a cancer diagnosis, a heart condition diagnosis, etc.). Any of the patient information may be used to sort a patient into a patient group. The CT simulator can then optimize scan parameters for a protocol for a patient group using scan data from one or more patients in an identified patient group. A patient group may be based on size (e.g., small adult, medium adult, large adult, pediatric, etc.), clinical group, and/or diagnostic information (e.g., heart condition). Additionally, the example CT simulator can also simulate how projected IQ measure and dose changes across groups and can plot a distribution across, for example, patients of different sizes.

The user may also access a Digital Imaging and Communications in Medicine (DICOM) image library 214 via an image provider service 216. The DICOM images may be used in addition to or in place of a scout scan of a patient as an input for the CT simulator 104. The DICOM image library 214 which may each include a plurality of DICOM images acquired using a CT system. DICOM image library 214 may be a non-limiting example of, or included in, a picture archiving and communications system (PACS). In particular, DICOM image library 214 may include a plurality of scout images. The CT simulator 104 may receive a scout image from the DICOM image library 214, and process the scout image to calculate an estimated dose (CTDlvol and DLP) and/or estimate an image quality (IQ) measure of an image reconstructed from projection data acquired from a patient of scout image during a scan, as described in greater detail below.

The example CT simulator 104 includes at least one dose algorithm service 218. The dose algorithm service 218 may include a dose algorithm equivalent to what is run on a CT imaging system. The dose algorithm uses the inputs provided by the user and outputs a dose or dose curve. The dose algorithm service 218 may include instructions for estimating a projected dose of a scan using the selected scan protocol, in accordance with scan settings output by the CT simulator 104 based on the protocol. Dose algorithm service 218 may model performance of a CT scanner and estimate the projected dose. In particular, dose algorithm service 218 may include instructions that, when executed by processor, cause the simulated CT system 100 to conduct one or more of the steps of method 300.

The example CT simulator 104 may further include an IQ algorithm service 222. The IQ Algorithm Service 222 may include one ore more algorithms that may be used to determine the parameters of a scan protocol optimized to balance dose with IQ for a patient or patient group. The example algorithms may mirror what is run on a CT imaging system 201. The example algorithms may include a noise based automatic exposure control (AEC) algorithm to determine an amount of noise that may be expected in an image resulting from the scan using the scan protocol and selected parameters for the patient or patient group. A dose based AEC algorithm may be run to minimize dose for a patient when using a selected scan protocol. The example CT simulator may also include a patient specificity service 220, provides information for a patient-related parameters determined by the CT simulator. For example, an auto-prescription algorithm may be used to determine a set of scan settings used for scanning a patient in accordance with a protocol. That is, a user of simulated CT system 100 may specify a protocol to use for a scan via user input device, and one or more AI models of the plurality of AI models may be used to configure the CT scanner based on the protocol. Additionally, an algorithm of the patient specificity service 220 may determine a scan range (e.g., a scan start position, a scan end position) of the patient or patient group. The example one or more algorithms of the dose algorithm service 218, the IQ algorithm service 222, and the patient specificity service 220 together determine the parameters to be used with a selected protocol to optimize the balance between dose and IQ for a resultant image from a CT scan.

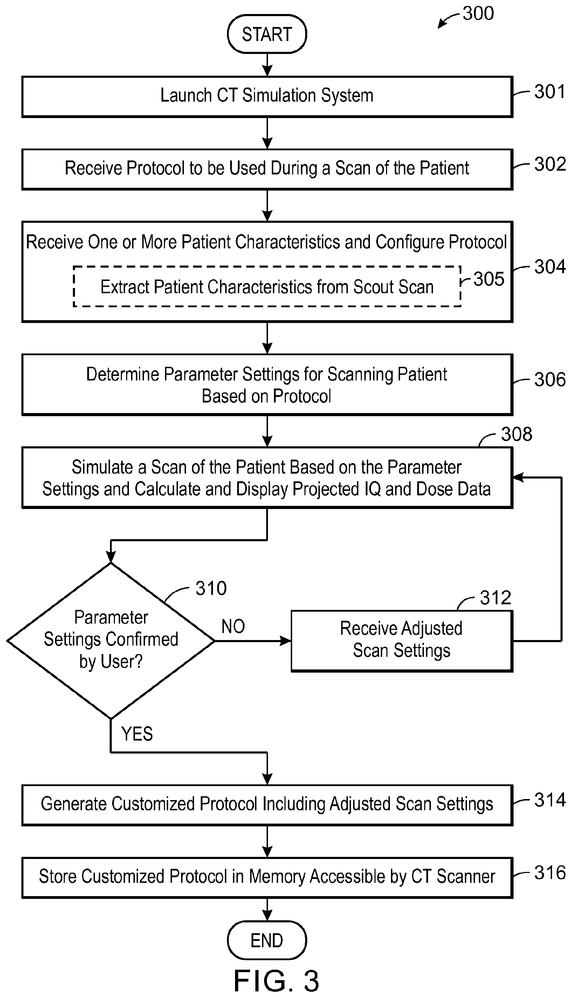

Turning now to FIG. 3, an exemplary method 300 is shown for simulating a diagnostic scan and previewing projected dose and image quality (IQ) data for a patient, based on a selected protocol and provided patient data. The method 300 may be performed by the simulated CT system 100. Operations of method 300 may be stored in non-transitory memory of the simulated CT system 100 and executed by a processor of the simulated CT system. It should be appreciated that in various examples, one or more steps of method 300 may be performed a plurality of times, for example, in an iterative fashion.

The method 300 begins at 301, where the method 300 includes launching the CT simulator 104. In some examples, launching the simulated CT system includes using the browser 202 of the CT simulator 104 to launch a user interface (e.g., via the scan simulator user interface files 203). In various examples, the CT simulator 104 may be installed on a computing device of a user, such as an operator of the CT imaging system (e.g., a radiologist, or medical physicist). Alternatively, the CT simulator 104 is stored on a server accessible by the operator workstation 102 used by the radiologist and/or medical physicist. The CT simulator 104 may be launched by the user prior to, and/or in preparation for performing a CT imaging scan on a patient. When the CT simulator 104 is launched, a GUI of the CT simulator 104 may be displayed on a display screen of the simulated CT system 100 (e.g., a display of the operator workstation 102).

At 302, method 300 includes receiving a protocol to be used during a subsequent diagnostic scan of the patient. The protocol may be selected from a plurality of protocols (e.g., from a protocol database 206 available to the user, which may correspond to protocols available to the CT imaging system 201.

At 304, method 400 includes receiving one or more patient characteristics of the patient, which may be used to configure the received protocol for the patient or patient group. The one or more patient characteristics may be received from a user of the CT simulator 104 via the GUI. For example, the one or more patient characteristics may include a size of the patient, or a body mass index (BMI) of the patient, or a water equivalent diameter (WED) of the patient, or a different physical characteristic of the patient or patient group.

In various examples, a patient characteristic may be extracted from a patient image, or a set of measurements taken of the patient. For example, at 305, method 300 may include extracting the patient characteristic (such as the patient size) from a localizer (e.g., scout) image of a patient. The scout image may be received from a storage location on the computing device, or a storage device coupled to the computing device, such as an image repository of a CT imaging system (e.g., DICOM image library 214 of FIG. 2). For example, the user may specify the scout image to be received via the GUI. In some embodiments, the user may browse or search a plurality of scout images stored at one or more locations in the computing device or on a network of the computing device using the GUI, and select a desired scout image to load into the CT simulation system. In other examples, the patient characteristic may be extracted from a 3D camera image of the patient taken during a scout scan, or from an electrocardiogram (EKG) recorded from the patient, or a different type of patient data acquired from the patient.

The CT simulator 104 GUI (e.g., the GUI) may include sets of controls that are the same as or similar to sets of controls of a CT imaging system GUI displayed in an operator console of the CT imaging system (e.g., operator console) and used to perform a scan on a patient. That is, the GUI may provide similar functionalities as the CT imaging system GUI, which may be accessed via controls having a similar appearance as controls of the CT imaging system GUI, in a similar manner as the CT imaging system GUI. For example, the user may load a localizer image (e.g., a scout scan), select a protocol for performing a diagnostic scan, request recommended scan parameters in accordance with the protocol, and/or set a plurality of scan settings manually using controls of the GUI that are similar to corresponding controls of the CT imaging system GUI. In this way, a familiarity of controls and UI design of the CT simulator 104 system GUI may reduce a learning curve for using the CT simulation system and facilitate a rapid and easy use of the CT simulation system by the user.

At 306, method 300 includes determining parameter settings (also referred to herein as scan settings) for scanning the patient, based on the received protocol and the one or more patient characteristics. In various embodiments, the parameter settings may be determined by one or more algorithms, such as the dose algorithm service, the noise based AEC algorithm, the dose based AEC algorithm, the smartplan algorithm, the auto prescription algorithm, the GSI assist algorithm, the autogating algorithm, and the kV assist algorithm, which may be included in the patient specificity service 220 or the IQ algorithm service 222 of FIG. 2. For example, the algorithms may include an AEC system, a scan range selection system, etc. That is, the one or more algorithms may take as input the received scan protocol and additional patient characteristics, and may output one or more sets of scan parameters for performing the scan in accordance with the protocol. The one or more sets of scan parameters may be displayed on the display screen to be viewed by the user.

At 308, method 300 includes simulating a diagnostic scan of the patient in the CT simulation system, based on the one or more sets of scan parameters. When the scan is simulated, the CT simulation system may calculate projected (e.g., estimated) dose data for the simulated scan. The projected dose may be an estimate of a dose for applied scan settings or parameters (e.g., kV, mA, rotation time, collimation, helical pitch, etc.). Calculating the projected dose data may include, for example, calculating a projected CTDIᵥₒₗ, a projected DLP, and/or a size-specific dose estimate (SSDE) for the scan. The projected dose data may also include a projected exposure duration for the scan protocol and settings or parameters. The projected dose data may be used, along with patient characteristics such as patient size, an anatomical region being scanned, and/or other factors to estimate an effective dose of the scan for the patient.

The applied scan settings or parameters may be inputted into the CT scanner model, and the CT scanner model may output the projected dose data, projected IQ data, and/or other projected data. In some embodiments, the CT scanner model may rely on a set of pre-measured values for specific scan parameters, and may apply a set of correction factors based on actual applied settings of the scan as modified by the user. The projected IQ data may be generated from the projected dose data, and may be used to display an exemplary image corresponding to the projected dose data. For example, the projected IQ data may include a projected noise index (NI) of the scan, and the projected NI may be used to generate the exemplary image. The projected dose and IQ data may be identical to what would be seen in a clinical setting, if the diagnostic scan were performed on the patient.

The projected dose, IQ data, and image may be displayed on a display screen of the operator workstation via the CT simulator user interface. An exemplary CT simulator user interface is described below in reference to FIG. 5.

The projected NI of the scan may be inversely proportional to the projected dose index (CTDIᵥₒₗ). That is, as the projected dose increases, the projected NI may decrease (e.g., increasing the IQ). Thus, prior to performing the diagnostic scan on the patient, the user may use the CT simulator 104 (e.g., via the CT simulator UI) to view examples of different IQs that may be achieved at different doses, to customize the scan parameters to the patient and achieve a desired IQ measure, while maintaining the dose within a desired or acceptable range of doses.

At 310, method 300 includes determining whether the scan parameter settings are confirmed by the user, using the CT simulator UI. If at 310 it is determined that the parameter settings are not confirmed, method 300 proceeds to 312. At 312, method 300 includes receiving adjusted scan parameters, and method 300 proceeds back to 308 to re-simulate the scan of the patient based on the adjusted scan parameters. For example, the one or more scan parameter settings may be adjusted by the user via individual controls for each parameter setting in the CT simulator UI, as described below in reference to FIG. 5. After the scan parameters are adjusted, updated IQ measure and dose data may be displayed in the CT simulation GUI, in real time. In this way, the user may test various different adjustments to the parameter settings to determine a combination of settings that achieves the desired image quality, at an acceptable dose.

Alternatively, if at 310 it is determined that the parameter settings are confirmed, it may be inferred that the parameter settings are acceptable to the user, whereby method 300 proceeds to 314. At 314, method 300 includes modifying the protocol to generate a customized protocol for the patient, where the customized protocol includes the adjusted scan settings. At 316, method 300 includes storing the scan parameter settings in a memory accessible by a CT imaging system 201.

The stored parameter settings may be exported to the CT imaging system, to be used in a subsequent diagnostic scan of the patient or patient group. By performing the diagnostic scan using the adjusted parameter settings determined using the simulated CT system 100, rather than recommended parameter settings automatically generated based on the protocol, a target IQ measure of an image reconstructed from projection data acquired during the diagnostic scan may more accurately achieved, using a lower radiation dose than generated using the recommended parameter settings. In this way, a specific IQ/dose balance may be customized to the patient in an efficient and rapid manner. Additionally, because the simulated CT system 100 is implemented as a stand-alone product and does not rely on a processor of the CT imaging system, the customized IQ/dose balance may be determined offline, meaning, without using resources of the CT imaging system. For example, the simulated CT system 100 may be used by a plurality of users in parallel, while the CT imaging system is being used to perform diagnostic scans on other patients, or the simulated CT system 100 may be used while the CT imaging system is in an unlaunched or unpowered state. As a result, the resources of the CT imaging system may be available for other tasks during use of the simulated CT system 100, increasing an efficiency and functionality of the CT imaging system.

Because the customized parameter settings are determined using the simulated CT = system in an offline fashion, meaning, not relying on the CT imaging system, the CT imaging system may be maintained available for diagnostic scans for a greater number of patients than in an alternative scenario where the simulated CT system is not available. In the alternative scenario, the operator may monopolize the CT imaging system for a longer period of time, due to performing multiple diagnostic scans on patients to achieve an image with a desired quality.

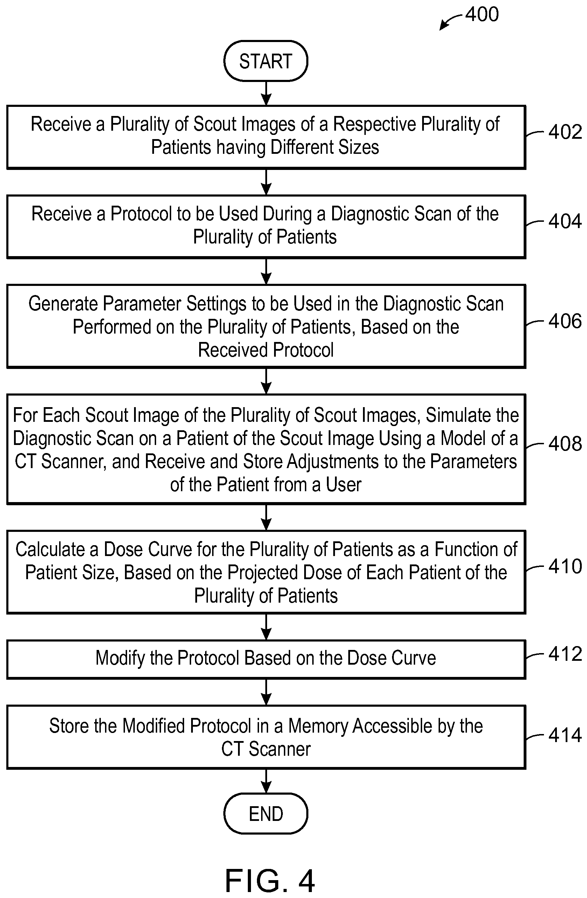

Referring now to FIG. 4, an exemplary method 400 is shown for modifying a protocol for a diagnostic scan for a plurality of patients to achieve a smoother dose curve as a function of patient size, using a simulated CT simulation system 100. The simulated CT system 100 allows a user to preview projected dose and image quality (IQ) data for patients of different sizes, as described above in reference to FIG. 3. Operations of method 300 may be stored in non-transitory memory of the simulated CT system 100 and executed by a processor of the simulated CT system 100.

Method 400 begins at 402, where method 400 includes receiving a plurality of scout images of a respective plurality of patients, where the patients have a range of different sizes. The plurality of scout images may be received from one or more repositories of DICOM images, such as DICOM image library.

At 404, method 400 includes receiving a protocol (e.g., an initial protocol) to be used during a diagnostic scan of the plurality of patients. The initial protocol may be selected by a user of the simulated CT system 100, via a GUI of the CT simulation system, such as the GUI shown in FIG. 5.

At 406, method 400 includes generating parameter settings to be used in the diagnostic scan performed on the plurality of patients, based on the received initial protocol. A first portion of the parameter settings may be configured based on a patient characteristic, such as a size of the patient, where the patient size is calculated from the scout images. A second portion of the parameter settings may be generated by one or more algorithms, such as the automated scan prescription algorithms.

At 408, method 400 includes, for each scout image of the plurality of scout images, simulating the diagnostic scan on a patient of the scout image. When the diagnostic scan is simulated, projected dose and IQ data for the diagnostic scan may be displayed on a display device, such as display device. The user may view the projected dose and IQ data to determine whether the projected IQ measure of an image reconstructed from the diagnostic scan exceeds a threshold quality. Method 400 also includes receiving adjustments to the parameter settings for the patient from the user. That is, for each scout image, the user may adjust one or more parameter settings via the GUI, and view updated projected dose and IQ data for a simulated diagnostic scan of the corresponding patient. The user may adjust the one or more parameter settings various times, in an iterative process, to achieve a desired IQ measure at an acceptable dose. When the parameter settings that achieve the desired IQ measure at the acceptable dose are determined by the user, the user may confirm the parameter settings via a control element of the UI. When the parameter settings for the patient are confirmed, the parameter settings, the resulting projected IQ measure, and the corresponding projected dose for the patient may be stored in a non-transitory memory of the system.

At 410, method 400 includes calculating a dose curve for the plurality of patients as a function of patient size, based on the projected dose of each patient of the plurality of patients. That is, after diagnostic scans have been simulated for each patient of each scout image of the plurality of scout images, with corresponding parameter setting adjustments collected for each simulated diagnostic scan, the plurality of patients may be ordered according to patient size, and a curve may be fitted to the projected doses for each patient for the size of the patient.

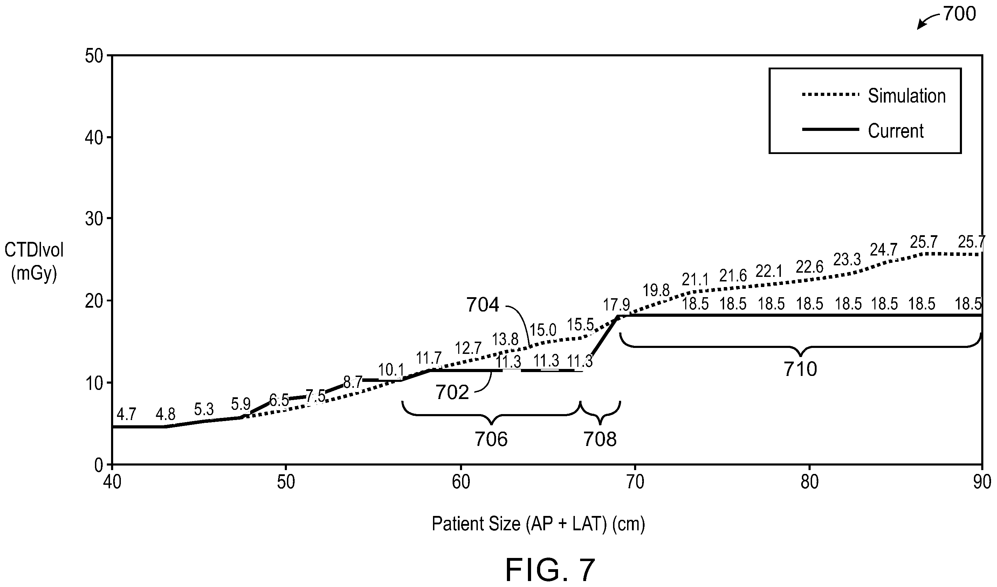

Referring briefly to FIG. 7, an exemplary dose curve graph 700 is shown, which shows dose as a function of patient size for simulated and actual patient data with respect to diagnostic scan. The patient size may be calculated from a scout image, as described above, and may include an anterior-posterior (AP) or front-to-back size of the patient, and a lateral (LAT) side-to-side size of the patient, measured in centimeters, for example. A dose applied to a patient is indicated on a y axis of dose curve graph 700, in CTDIᵥₒₗ, and a size of the patient corresponding to the dose is indicated on an x axis of dose curve graph 700. Dose curve graph 700 includes two plots of dose curves. A first dose curve 702 is plotted based on a first protocol, with a first set of parameter settings indicated by the first protocol for each patient. A second dose curve 704 is plotted based on a second protocol with simulated (e.g., projected) dose data associated with each patient, where the dose data is generated as a result of simulating the diagnostic scan of the patient using the CT simulation system. In particular, the dose data of second dose curve 704 is based on adjusted parameter settings generated as described in methods 300 and 400. In other words, the second protocol is a version of the first protocol that has been customized for each patient by a user of the CT simulation system. On each dose curve, an amount of the dose measured in mGy is shown for each patient.

As can be seen in FIG. 7, first dose curve 702 does not show a smooth evolution of dose as a function of patient size. In particular, a first portion 706 of first dose curve 702 indicates that an equivalent dose (11.3 mGy) is provided to patients who have sizes ranging from approximately 58 to 67 cm. A second portion 708 of first dose curve 702 indicates that doses increase steeply for patients who have sizes ranging from approximately 67 to 69 cm. A third portion 710 of first dose curve 702 indicates that an equivalent dose (18.5 mGy) is provided to patients who have sizes ranging from approximately 69 to 90 cm. Thus, if diagnostic scans are performed using the first protocol, patients with decreasing or increasing sizes over a course of treatment may be administered doses that are not tailored well to patient size, which may result in suboptimal image quality (if the patient does not receive enough dose) or a dose that is higher than necessary to achieve the desired image quality.

In contrast, second dose curve 704 shows a smoother evolution of dose as a function of patient size than first dose curve 702, where different doses are consistently applied to patients of different sizes, in similar increments. Therefore, if diagnostic scans are performed using the second, customized protocol, the patients with decreasing or increasing sizes over the course of treatment may be administered doses that are more precisely tailored to patient size, leading to a more optimal trade-off between radiation dose and image quality.

Returning to FIG. 4, at 412, method 400 includes modifying the protocol based on the calculated dose curve. That is, when appropriate adjusted parameter settings are collected from the user for each scout image/patient that generate a dose curve with desired properties, such as the smooth evolution of dose as a function of patient size shown by second dose curve 704 of FIG. 7, the protocol may be modified to include the appropriate adjusted parameter settings. The modified protocol may be used to set parameter settings for future diagnostic scans of patients of varying sizes, rather than the initial protocol. The modified protocol may specify parameter settings for each of the patients of the varying sizes that more smoothly adjust doses applied to the patients as a function of size than the initial protocol.

At 414, method 400 includes storing the modified protocol in a memory accessible by the CT scanner. During a subsequent diagnostic scan, the modified protocol may be retrieved from the memory and applied by an operator of the CT scanner. Method 400 ends.

FIG. 5 shows an exemplary GUI 500 of a simulated CT system100, which may be used in methods 300 and 400 described above to simulate a diagnostic scan of a patient on a CT scanner. It should be appreciated that GUI 500 is provided for illustrative purposes and not for limitation, and in other embodiments GUI 500 may include a greater or lesser number of components, or different components, without departing from the scope of this disclosure.

GUI 500 may include a top bar 502, which may provide space for various high-level controls, such as login or exit functions, user profile and/or other high-level menu options, and the like. In some examples, GUI 500 may offer a tabbed view, where data of different scans or patients may be presented in a plurality of tabs accessible from top bar 502.

In the depicted embodiment, GUI 500 includes a first display panel 510, a second display panel 520, a third display panel 522, a fourth display panel 524, and a fifth display panel 526, which may display various types of patient data. Specifically, first display panel 510 includes a progress bar, which may display progress of a scan being performed. Second display panel 520 may include general, high-level information about a selected patient or case. For example, a user of GUI 500 may select a patient via a menu accessed via top bar 502, which may display a plurality of patients of a health care system, for example. Data of the selected patient may be retrieved from a storage location and displayed in GUI 500. The storage location may be a memory of the simulated CT system 100, or a storage location of a different system accessible via a network. The high-level information may include, for example, identifying information of the patient, a health issue of the patient, an availability of images, lab results, historical data such as procedures performed on the patient, and the like. Second display panel 520 may also include control elements, such as one or more buttons for performing various tasks or viewing different types of patient data. In particular, second display panel 520 may include a control element for initiating a simulation of a diagnostic scan on the patient, as described above in reference to FIG. 3.

Third display panel 522 and fourth display panel 524 may be used to display parameter settings associated with a selected protocol. For example, the user may select a protocol to be used in a future diagnostic scan of the patient. In some embodiments, the protocol may be selected from a pop-up menu displayed as a result of the user selecting the control element for initiating the simulation of the diagnostic scan. When the protocol is selected, parameter settings associated with the protocol may be displayed in one or both of third display panel 522 and fourth display panel 524. The parameter settings may be configured or determined by one or more algorithms, such as the automated scan prescription algorithms. In some examples, the user may select the one or more algorithms manually using control elements of GUI 500, while in other examples, the one or more algorithms may be selected automatically. The one or more algorithms may take patient data and the protocol as input, and output a respective set of parameter settings for the protocol. The patient data may include one or more patient characteristics, such as patient size. In some embodiments, the patient data may include or be extracted from a scout image obtained from the patient at a previous time. The scout image may be displayed in a seventh display panel 530 and/or an eighth display panel 532 of GUI 500, in some examples.

The scan parameters generated by the one or more AI models and displayed in third display panel 522 and/or fourth display panel 524 may be editable (e.g., adjustable) by the user. As described above, the user may adjust one or more of the scan parameters for the selected protocol. In particular, the user may initiate a simulation of a diagnostic scan on the patient using the parameter settings of the protocol, and view projected dose and IQ data for the simulated diagnostic scan based on the parameter settings. The user may then adjust one or more of the scan parameters, and view updated projected dose and IQ data for the simulated diagnostic scan based on the adjusted parameter settings. In this way, the user may iteratively determine customized parameter settings to be applied as part of the protocol for the patient in the future diagnostic scan. The projected dose and IQ data may be displayed in fifth display panel 526. In some cases, visualizations of the updated parameter settings may additionally or alternatively be displayed in one or more of the display panels 522, 524, 526, 530, and 532. For example, the user may adjust a starting and ending position of the diagnostic scan, and the adjusted starting and ending position of the diagnostic scan may be visualized on the scout image displayed in display panels 530 and 532.

An example settings summary display panel may be displayed in second display panel 520 of GUI 500, for example. Settings summary display panel includes various categories of parameter settings associated with a selected protocol, where individual settings in each category are summarized. For example, anatomy selection parameter settings, kV and mA control parameter settings, contrast parameter settings timing parameter settings, scan type parameter settings, coverage speed parameter settings, primary reconstruction parameter settings, and gating and trace parameter settings are summarized in the display panel 520. The settings summary may be displayed in a same or similar manner as the parameter settings are displayed to an operator of a CT scanner via an operator console of a CT imaging system. Thus, the display of the parameter settings in settings summary display panel 520 may be familiar to the user of GUI 500.

An exemplary projected dose display panel may be displayed in fifth display panel 526 of GUI 500, in various examples. Projected dose display panel 525 may display projected dose data for a simulated diagnostic scan initiated via GUI 500. The projected dose data may be based on parameter settings of the selected protocol, or adjusted parameter settings generated by the user. In the depicted embodiment, the projected dose data includes an overall projected dose value (e.g., 101.39), which may be generated from various dose data components. The dose data components may include, for example, a CTDIᵥₒₗ value measured in mGy; a DLP dose value measured in mGy*cm; a dose efficiency value that indicates a percent of the dose used to generate the image; and a size in cm of a CTDI phantom used when calculating CTDI and dose efficiency, where the phantom may be a Body phantom comprising a 32 cm cylindrical phantom, or a Head phantom comprising a 16 cm cylindrical phantom.

The projected dose value is a measure of the dose to the phantom for the applied scan settings (e.g., kV, mA, rotation time, collimation, helical pitch, etc.), and may be calculated by looking up a set of pre-measured values for specific settings, and then applying a set of correction factors based on the actual applied settings of the scan, as described above.

If the user is satisfied with the projected IQ measure and the projected dose, the user may select a control element to confirm the parameter settings displayed in GUI 500. If the user is not satisfied with the projected IQ measure and the projected dose, the user may adjust one or more of the parameter settings, and re-simulate the diagnostic scan to obtain different projected IQ measure and dose data. The user may simulate the diagnostic scan various times with different parameter settings to determine ideal parameter settings for the patient and the protocol. When the ideal parameter settings are obtained, the user may confirm the settings via control element. When the user selects control element, the selected protocol may be modified to include the adjusted parameter settings, thereby generating a customized protocol for the patient. The customized protocol may be used during the future diagnostic scan of the patient via the CT scanner.

An exemplary visualization of a starting point and an ending point of a simulated diagnostic scan, according to an embodiment may be displayed on a scout image displayed in GUI 500 of FIG. 5, for example, in seventh display panel 530 and eighth display panel 532. When a protocol is selected by the user, a selected portion of an anatomy of the patient that will be scanned in a diagnostic scan may be indicated by a first box on a first view of the scout image displayed in seventh display panel 530, and a second box on a second view of the scout image displayed in eighth display panel 532. In some examples, the example patient specificity service 220 of FIG. 2 may be used to identify the selected portion of the anatomy of the patient. A starting point of the diagnostic scan may be indicated by an upper line of first box, and by a left-most line of second box. An ending point of the diagnostic scan may be indicated by a lower line of first box, and by a right-most line of second box.

The user may adjust starting point and ending point parameters of the simulated diagnostic scan. When the user edits the starting point and ending point parameters, the selected portion of the anatomy of the patient that will be scanned in the diagnostic scan may be updated in the visualization, where the updated selected portion of the anatomy is indicated by first box on the first view of the scout image displayed in seventh display panel 530, and a second box on the second view of the scout image displayed in eighth display panel 532. An updated starting point of the diagnostic scan may be indicated by an upper line of first dotted box, and by a left-most line of second dotted box. An ending point of the diagnostic scan may be indicated by a lower line of first dotted box, and by a right-most line of second dotted box. Thus, visualization may visualize an impact of adjustments to the starting and ending point parameters made textually via anatomy selection display panel on the scout image, which may make it easier for the user to identify ideal starting and ending point parameters for the diagnostic scan.

In some embodiments, upper line, lower line, left-most line, and right-most line may be selectable, such that the user may interactively adjust a position of one or more of upper line, lower line, left-most line, and right-most line to reflect a desired starting and/or ending point. When the user interactively adjusts the position(s), corresponding starting and/or ending point parameters may be automatically updated in the simulated CT system and in anatomy selection display panel.

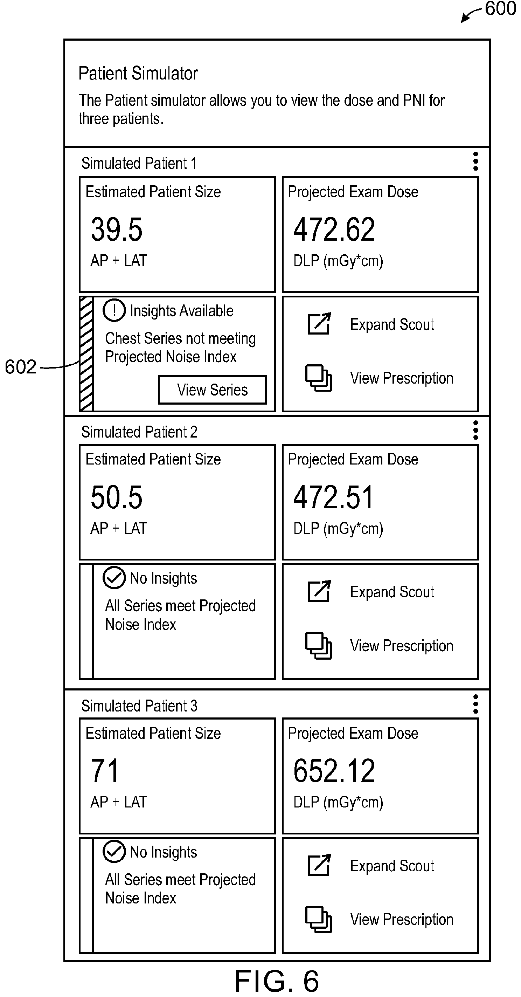

FIG. 6 depicts another example user interface 600 that may be displayed to a on an operator workstation 102 when using the simulated CT system 100. The example user interface 600 includes estimated patient size and projected exam dose for a plurality of patients simultaneously. The example user interface 600 depicts three simulated patients at three different sized (e.g., small adult, medium adult, and large adult). However, any number of simulated patients may be depicted. In the illustrated example, there is an insight available for simulated patient 1. In such cases, the insights box 602 may be depicted as a different color than when there are no insights available. Example insights include suggested adjustments to a smartplan for the patient, such as adjusting the start or stop location for the scan on the body of the patient. Another example insight includes adjusting the kV to meet the recommended noise requirements.

The technical effect of simulating a diagnostic scan of a patient performed by a CT imaging system, and allowing a user of a simulated CT system to adjust parameter settings of a selected protocol for the diagnostic scan and view projected dose and image quality data of the simulated diagnostic scan, is that a customized protocol can be generated for the patient that results in a reconstructed image of a desired image quality measure without wasting operator time and resources of the CT imaging system in repeated diagnostic scans.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative and should not be construed to be limiting in any manner.

## Claims

1. A computer-implemented method for a simulated computed tomography (CT) system, the method comprising:
receiving, via a user interface, a selected scan protocol;
receiving, via the user interface, one or more characteristic of a patient or patient group;
determining, using a CT simulator, parameter settings for scanning the patient or patient group;
determining, using the CT simulator, a projected image quality measure or a projected dose; and
generating a customized scan protocol for the patient or patient group.

2. The method of claim 1, further including displaying the projected image quality measure and dose on a display of the simulated CT system.

3. The method of claim 1, further including launching a CT simulator on an operator workstation, wherein the operator workstation is remote from a CT imaging system.

4. The method of claim 1, wherein receiving one or more characteristics of a patient or patient group includes extracting characteristics from a scout scan.

5. The method of claim 1, wherein determining the projected image quality measure and dose includes simulating a scan of the patient or patient group based on the selected scan protocol, characteristic and parameter settings.

6. The method of claim 1, further comprising receiving confirmation from a user that the parameter settings are acceptable.

7. The method of claim 6, wherein when the parameter settings are not acceptable, receiving adjusted parameter settings via the user interface.

8. The method of claim 1, further comprising saving the customized scan protocol in a memory accessible by the CT system.

9. A simulated computed tomography (CT) system, comprising:
an operator workstation;
a CT simulator accessible via the operator workstation, the CT simulator configured to:
receive, via a user interface displayed on the operator workstation, a selected scan protocol;
receive, via the user interface, one or more characteristic of a patient or patient group;
determine parameter settings for scanning the patient or patient group;
determine a projected image quality measure and dose; and
generate a customized scan protocol for the patient or patient group.

10. The system of claim 9, wherein the CT simulator is further configured to display the projected image quality measure and dose via the user interface on the operator workstation.

11. The system of claim 1, wherein the received one or more characteristics of a patient or patient group are extracted characteristics from a scout scan.

12. The system of claim 1, wherein the projected image quality measure and dose are determined by simulating a scan of the patient or patient group based on the selected scan protocol, characteristic and parameter settings.

13. The system of claim 1, wherein the CT simulator is further configured to receive confirmation from a user that the parameter settings are acceptable.

14. The system of claim 13, wherein when the parameter settings are not acceptable, the CT simulator is further configured to receive adjusted parameter settings via the user interface.

15. The system of claim 1, further comprising a CT system, wherein the customized protocol is saved in a memory accessible by the CT system.
